(19) Europäisches Patentamt
European Patent Office
Office européen des brevets

(11) Veröffentlichungsnummer: **0 572 440 B1**

(12) **EUROPÄISCHE PATENTSCHRIFT**

(45) Veröffentlichungstag der Patentschrift: **02.08.95**

(51) Int. Cl.⁶: **C07D 401/04**, C07C 225/16, A01N 43/54

(21) Anmeldenummer: **92903932.9**

(22) Anmeldetag: **13.02.92**

(86) Internationale Anmeldenummer: **PCT/EP92/00311**

(87) Internationale Veröffentlichungsnummer: **WO 92/14726 (03.09.92 92/23)**

Die Akte enthält technische Angaben, die nach dem Eingang der Anmeldung eingereicht wurden und die nicht in dieser Patentschrift enthalten sind.

# (54) SUBSTITUIERTE PYRIDYLPYRIMIDINE, DEREN HERSTELLUNG, IHRE VERWENDUNG UND NEUE ZWISCHENPRODUKTE.

(30) Priorität: **23.02.91 DE 4105751**

(43) Veröffentlichungstag der Anmeldung: **08.12.93 Patentblatt 93/49**

(45) Bekanntmachung des Hinweises auf die Patenterteilung: **02.08.95 Patentblatt 95/31**

(84) Benannte Vertragsstaaten: **BE CH DE ES FR GB IT LI NL**

(56) Entgegenhaltungen:
**EP-A- 0 259 139**
**EP-A- 0 270 362**
**EP-A- 0 322 391**
**WO-A-91/07400**

(73) Patentinhaber: **BAYER AG**
**D-51368 Leverkusen (DE)**

(72) Erfinder: **HEINEMANN, Ulrich**
**Am Sonnenhang 1**
**D-5653 Leichlingen 2 (DE)**
Erfinder: **DEHNE, Heinz-Wilhelm**
**Krischer Strasse 81**
**D-4019 Monheim (DE)**
Erfinder: **DUTZMANN, Stefan**
**Kosenberg 10**
**D-4010 Hilden 1 (DE)**
Erfinder: **ERDELEN, Christoph**
**Unterbüscherhof 22**
**D-5653 Leichlingen 3 (DE)**

(74) Vertreter: **Schumacher, Günter, Dr. et al**
**Bayer AG**
**Konzernverwaltung RP**
**Patentabteilung**
**D-51368 Leverkusen**
**Bayerwerk (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 572 440 B1

## Beschreibung

Die Erfindung betrifft neue substituierte Pyridylpyrimidine, ein Verfahren zu ihrer Herstellung, ihre Verwendung als Schädlingsbekämpfungsmittel und neue Zwischenprodukte.

Es ist bekannt, daß bestimmte substituierte Pyridylpyrimidine, wie beispielsweise die Verbindung 2-(6-Phenyl-2-pyridyl)-4-chlor-6-methylpyrimidin oder die Verbindung 2-(6-Methyl-2-pyridyl)-4-(2-methylphenyl)-pyrimidin oder die Verbindung 2-(6-Methyl-2-pyridyl)-4-hydroxy-6-phenyl-pyrimidin fungizide Eigenschaften besitzen (vgl. z.B. EP-A 259 139 oder EP-A 270 362). Die Wirksamkeit dieser vorbekannten Verbindungen ist jedoch insbesondere bei niedrigen Aufwandmengen und Konzentrationen nicht in allen Anwendungsgebieten völlig zufriedenstellend.

Aus der WO-A 91-07 400, die erst nach dem Prioritätsdatum der vorliegenden Anmeldung publiziert wurde, sind Pyridylpyrimidine bekannt, in denen der Pyrimidin-Ring in der 4-Position durch einen über eine Alkylenbrücke verbundenen, gegebenenfalls substituierten Phenylrest substituiert ist. Es werden aber keine Stoffe dieses Typs offenbart, in denen die Alkylenbrücke verzweigt ist.

Im übrigen werden in der EP-A 0 322 391 bestimmte Phenylalkylamin-Derivate mit pharmakologischen Eigenschaften beschrieben. Entsprechende Verbindungen, in denen eine -CH = CH-Einheit zwischen Amino- und Carbonyl-Gruppe enthalten ist, werden jedoch nicht erwähnt.

Es wurden neue substituierte Pyridylpyrimidine der Formel

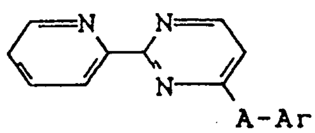

(I)

in welcher

A für einen Rest der Formel

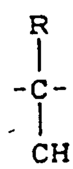 ; 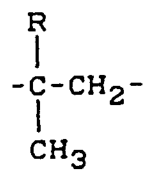 oder 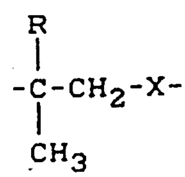

steht, wobei

- R jeweils für geradkettiges oder verzweigtes Alkyl mit 1 bis 4 Kohlenstoffatomen steht,
- X für Sauerstoff oder Schwefel steht und
- Ar für gegebenenfalls einfach bis fünffach, gleich oder verschieden substituiertes Phenyl steht, wobei als Substituenten infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen
  Alkylteilen, oder gegebenenfalls einfach bis fünffach, gleich oder verschieden durch Halogen und/oder geradkettiges oder verzweigtes Alkyl mit 1 bis 4 Kohlenstoffatomen substituiertes Phenyl.

gefunden.

Weiterhin wurde gefunden, daß man die neuen substituierten Pyridylpyrimidine der Formel (I) erhält, wenn man 2-Pyridylamidin-Salze der Formel

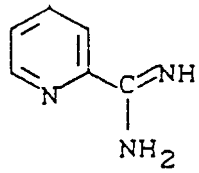 x HY (II)

in welcher

Y für ein Halogenid- oder Hydrogen-sulfatanion steht,

mit Enaminen der Formel

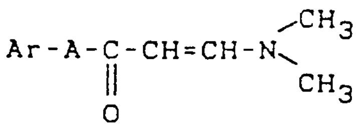 (III)

in welcher

Ar und A die oben angegebene Bedeutung haben,

gegebenenfalls in Gegenwart eines Verdünnungsmittels und gegebenenfalls in Gegenwart eines Reaktionshilfsmittels umsetzt.

Schließlich wurde gefunden, daß die neuen substituierten Pyridylpyrimidine der Formel (I) eine gute Wirksamkeit gegenüber Schädlingen besitzen.

Überraschenderweise zeigen die erfindungsgemäßen substituierten Pyridylpyrimidine der Formel (I) eine deutlich verbesserte Wirksamkeit z.B. gegen pflanzenpathogene Pilze im Vergleich zu den aus dem Stand der Technik bekannten substituierten Pyridylpyrimidinen, wie beispielsweise die Verbindung 2-(6-Phenyl-2-pyridyl)-4-chlor-6-methylpyrimidin oder die Verbindung 2-(6-Methyl-2-pyridyl)-4-(2-methylphenyl)-pyrimidin oder die Verbindung 2-(6-Methyl-2-pyridyl)-4-hydroxy-6-phenyl-pyrimidin, welche chemisch und wirkungsmäßig naheliegende Verbindungen sind.

Bevorzugt sind Verbindungen der Formel (I), bei welchen

A für einen Rest der Formel

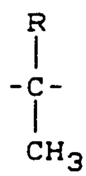 ; 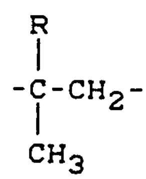 oder 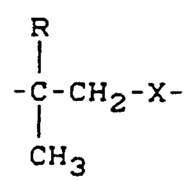

steht, wobei

R jeweils für Methyl oder Ethyl steht,

X für Sauerstoff oder Schwefel steht und

Ar für gegebenenfalls ein- bis dreifach, gleich oder verschieden substituiertes Phenyl steht, wobei als Substituenten infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Trifluormethylthio, Methoxycarbonyl, Ethoxycarbonyl, Methoximinomethyl, Ethoximinomethyl, Methoximinoethyl, Ethoximinoethyl oder gegebenenfalls ein- bis dreifach, gleich oder verschieden durch Fluor, Chlor, Brom, Methyl und/oder Ethyl substituiertes Phenyl.

Besonders bevorzugt sind Verbindungen der Formel (I), bei welchen

A für einen Rest der Formel

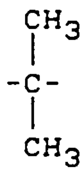 ; 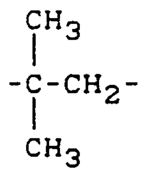 ; 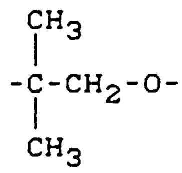 oder 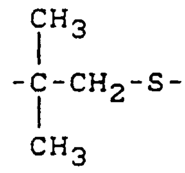

steht und

Ar für gegebenenfalls einfach oder zweifach, gleich oder verschieden substituiertes Phenyl steht, wobei als Substituenten infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Trifluormethylthio, Methoxycarbonyl oder Ethoxycarbonyl.

Im einzelnen seien außer den bei den Herstellungsbeispielen genannten Verbindungen die folgenden substituierten Pyridylpyrimidine der Formel (I) genannt:

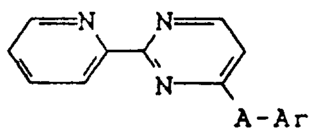

(I)

| A | Ar | A | Ar |
|---|---|---|---|
| 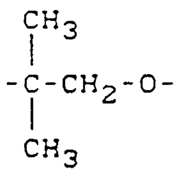 | 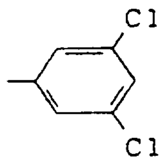 | 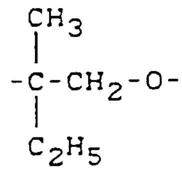 | 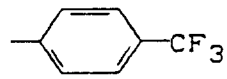 |
| 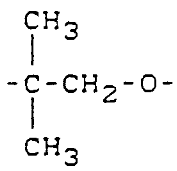 | 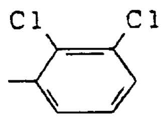 | 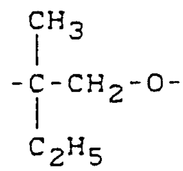 | 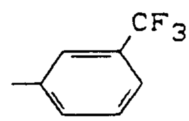 |
| 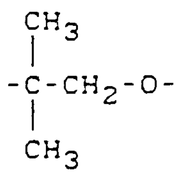 | 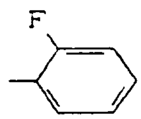 | 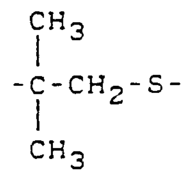 | 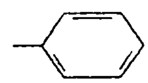 |
| 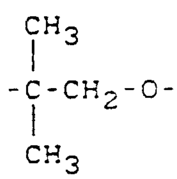 | 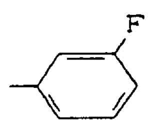 | 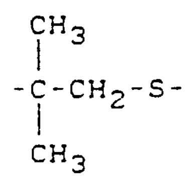 | 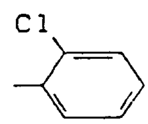 |
| 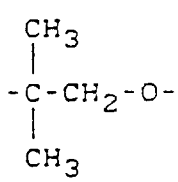 | 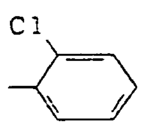 | 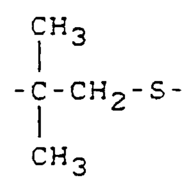 | 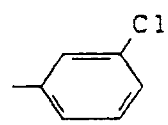 |
| 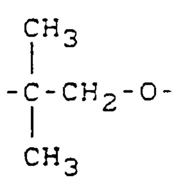 | 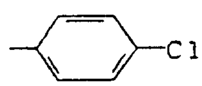 | 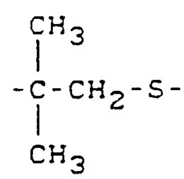 | 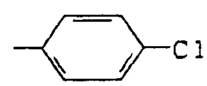 |
| 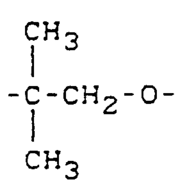 | 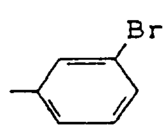 | 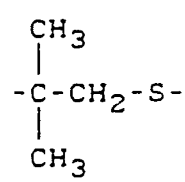 | 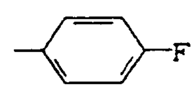 |

| A | Ar | A | Ar |
|---|---|---|---|
| 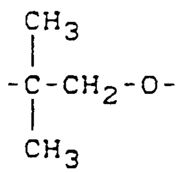 | 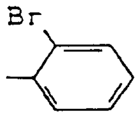 | 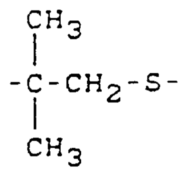 | 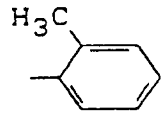 |
| 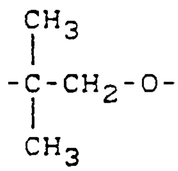 | 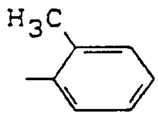 | 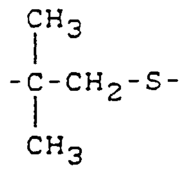 | 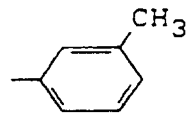 |
| 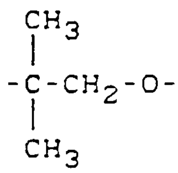 | 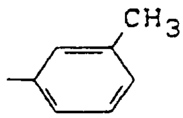 | 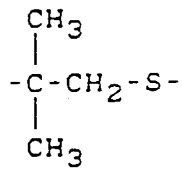 | 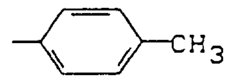 |
| 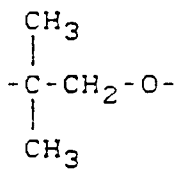 | 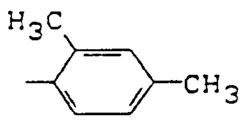 | 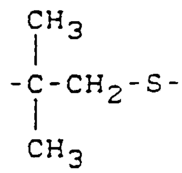 | 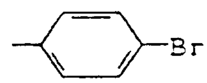 |
| 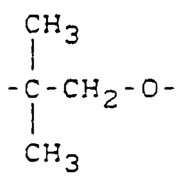 | 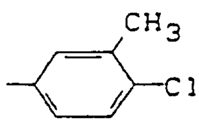 | 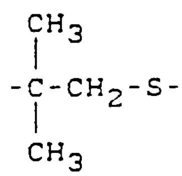 | 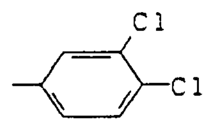 |
| 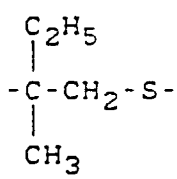 | 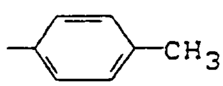 | 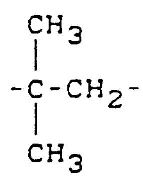 | 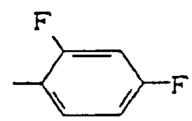 |
| 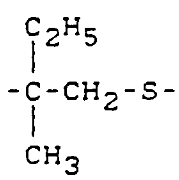 | 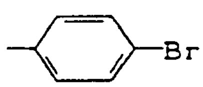 | 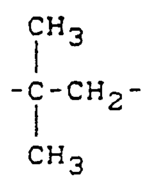 | 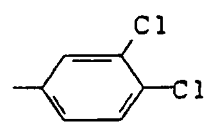 |

| A | Ar | A | Ar |
|---|---|---|---|
| 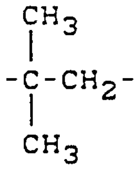 | 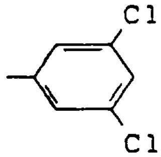 | 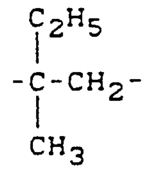 | 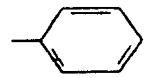 |
| 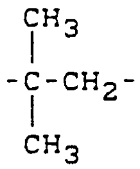 | 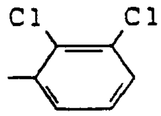 | 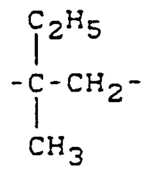 | 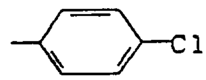 |
| 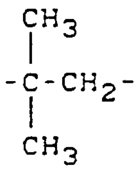 | 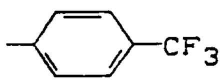 | 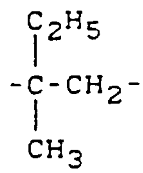 | 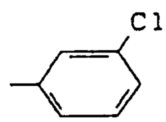 |
| 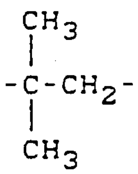 | 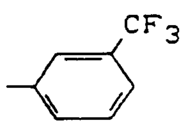 | 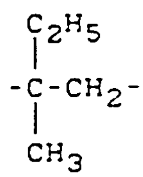 | 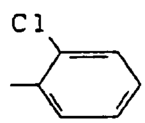 |
| 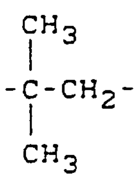 | 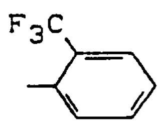 | 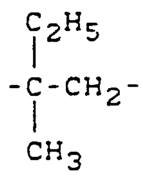 | 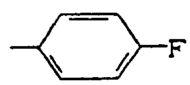 |
| 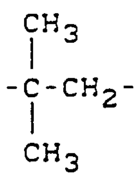 | 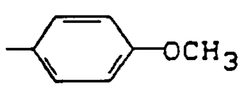 , | 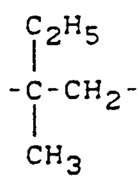 | 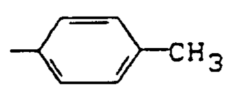 |
| 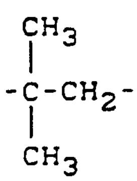 | 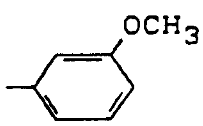 | 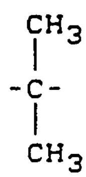 | 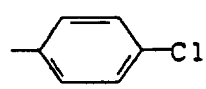 |

| A | Ar | A | Ar |
| --- | --- | --- | --- |
| 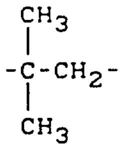 | 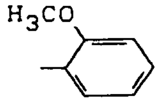 | 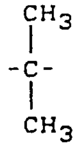 | 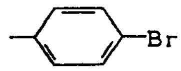 |
| 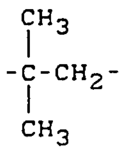 | 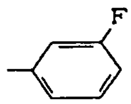 | 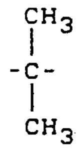 | 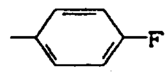 |
| 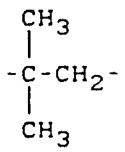 | 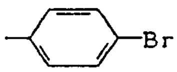 | 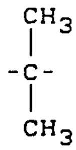 | 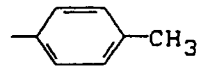 |
| 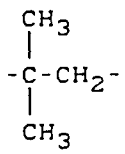 | 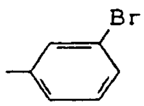 | 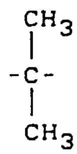 | 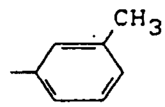 |
| 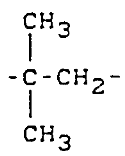 | 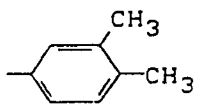 | 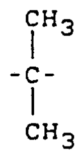 | 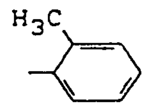 |
| 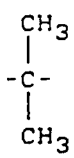 | 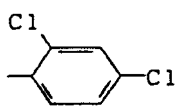 | 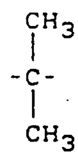 | 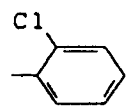 |
| 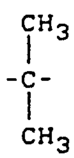 | 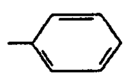 | 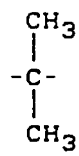 | 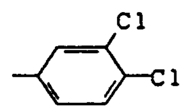 |
| 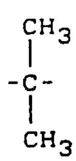 | 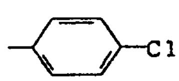 | 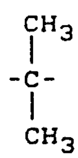 | 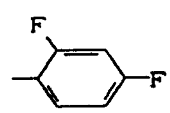 |
| 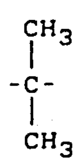 | 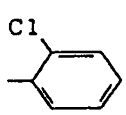 | | |

Verwendet man beispielsweise 2-Pyridylamidin-hydrochlorid und 1-Dimethylamino-4,4-dimethyl-5-(4-methylphenyl)-pent-1-en-3-on als Ausgangsverbindungen, so läßt sich der Reaktionsverlauf des erfindungsgemäßen Verfahrens durch das folgende Formelschema darstellen:

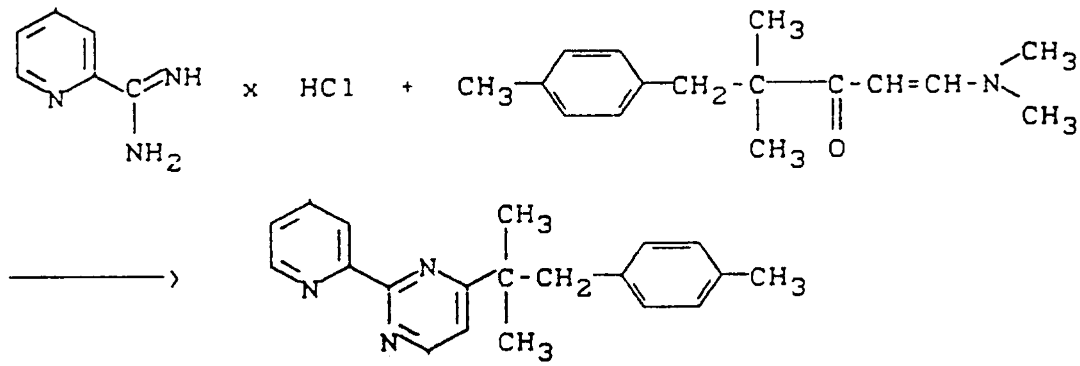

Die zur Durchführung des erfindungsgemäßen Verfahrens als Ausgangsstoffe benötigten 2-Pyridylamidin-Salze sind durch die Formel (II) allgemein definiert. In dieser
Formel (II) steht Y vorzugsweise für ein Chlorid-Anion, ein Bromid-Anion oder ein Hydrogensulfat-Anion. Y steht besonders bevorzugt für ein Chlorid-Anion.

Die 2-Pyridylamidin-Salze der Formel (II) sind bekannt (vgl. z.B. US-A 4.018.770 oder J. Am. Chem. Soc. 107, 5745-5754 [1985] oder J. Med. Chem. 33, 1230-1241 [1990]).

Die zur Durchführung des erfindungsgemäßen Verfahrens weiterhin als Ausgangsstoffe benötigten Enamine sind durch die Formel (III) allgemein definiert. In dieser Formel (III) stehen Ar und A vorzugsweise für diejenigen Reste, die bereits im Zusammenhang mit der Beschreibung der erfindungsgemäßen Stoffe der Formel (I) als bevorzugt für diese Substituenten genannt wurden.

Die Enamine der Formel (III) sind noch nicht bekannt und ebenfalls Gegenstand der Erfindung.

Man erhält sie, wenn man Ketone der Formel

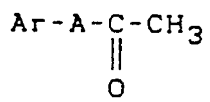 (IV)

in welcher

Ar und A die oben angegebene Bedeutung haben,

mit Formaldehyd-Derivaten der Formel

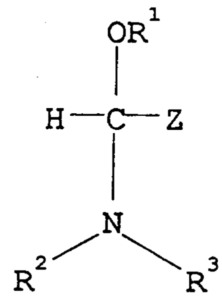 (V)

in welcher

Z für einen Rest $—OR^1$ oder für einen Rest

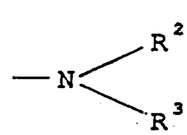

steht und

$R^1$, $R^2$ und $R^3$ unabhängig voneinander jeweils für geradkettiges oder verzweigtes Alkyl mit 1 bis 4 Kohlenstoffatomen stehen,

in annähernd äquimolaren Mengen gegebenenfalls in Gegenwart eines dipolar aprotischen Verdünnungsmittels, wie beispielsweise Dimethylformamid, N-Methylformanilid, N-Methylpyrrolidon, Dioxan, Ethylenglykoldimethylether, Ethylenglykoldiethylether oder Hexamethylphosphorsäuretriamid, vorzugsweise jedoch ohne Zusatz eines Verdünnungsmittels bei Temperaturen zwischen 20 °C und 160 °C, vorzugsweise zwischen 100 °C und 140 °C, umsetzt.

Ketone der Formel (IV) sind bekannt oder erhältlich in Analogie zu bekannten Verfahren (vgl. z.B. US-A 4.877.446; EP-A 301393; EP-A 296749; EP-A 289913; DE-A 3643851; GB-A 2120664; DE-A 3210725; DE-A 3021516).

Formaldehyd-Derivate der Formel (V) sind allgemein bekannte Verbindungen der organischen Chemie.

Die aufgeführten Definitionen der einzelnen Reste der Formel (I) gelten in entsprechender Weise auch für die Zwischen- und Vorprodukte.

Als Verdünnungsmittel zur Durchführung des erfindungsgemäßen Verfahrens kommen inerte organische Lösungsmittel infrage. Hierzu gehören insbesondere aliphatische, alicyclische oder aromatische, gegebenenfalls halogenierte Kohlenwasserstoffe wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester, Sulfoxide, wie Dimethylsulfoxid oder Alkohole, wie Methanol, Ethanol, sowie n- oder i-Propanol.

Das erfindungsgemäße Verfahren wird vorzugsweise in Gegenwart eines geeigneten Reaktionshilfsmittels durchgeführt. Als solche kommen alle üblicherweise verwendbaren anorganischen und organischen Basen infrage. Vorzugsweise verwendet man Alkalimetallhydride, -hydroxide, -amide, -alkoholate, -carbonate oder -hydrogencarbonate, wie beispielsweise Natriumhydrid, Natriumamid, Natriumhydroxid, Natriummethylat, Natriumethylat, Kalium-t-butylat, Natriumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens in einem größeren Bereich variiert werden. Im allgemeinen arbeitet man bei Temperaturen zwischen 20 °C und 140 °C, vorzugsweise bei Temperaturen zwischen 60 °C und 120 °C.

Das erfindungsgemäße Verfahren wird im allgemeinen bei Normaldruck durchgeführt. Es ist jedoch auch möglich, das erfindungsgemäße Verfahren unter erhöhtem oder vermindertem Druck durchzuführen.

Zur Durchführung des erfindungsgemäßen Verfahrens setzt man pro Mol an 2-Pyridylamidin-Salz der Formel (II) im allgemeinen 0,8 bis 1,5 Mol, vorzugsweise 1,0 bis 1,2 Mol an Enamin der Formel (III) und gegebenenfalls 1,0 bis 2,0 Mol, vorzugsweise 1,0 bis 1,5 Mol an Reaktionshilfsmittel ein.

Die Reaktionsdurchführung, Aufarbeitung und Isolierung der Reaktionsprodukte erfolgt nach allgemein üblichen Methoden (vgl. auch die Herstellungsbeispiele).

Die erfindungsgemäßen Wirkstoffe weisen eine starke Wirkung gegen Schädlinge auf und können zur Bekämpfung von unerwünschten Schadorganismen praktisch eingesetzt werden. Die Wirkstoffe sind insbesondere für den Gebrauch als Pflanzenschutzmittel insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannt:

Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus

(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten wie beispielsweise gegen den Erreger des echten Getreidemehltaus an Weizen oder Gerste (Erysiphe graminis) oder gegen den Erreger der Braunfleckenkrankheit der Gerste (Cochliobolus sativus) oder gegen den Erreger der Braunspelzigkeit des Weizens (Leptosphaeria nodorum) oder gegen den Erreger der Netzfleckenkrankheit der Gerste (Pyrenophora teres) oder gegen den Erreger des Getreideschneeschimmels (Fusarium nivale) sowie gegen andere Fusarium-Erreger wie beispielsweise Fusarium culmorum, außerdem zur Bekämpfung von Krankheiten im Reisanbau, wie beispielsweise gegen den Erreger der Reisfleckenkrankheit (Pyricularia oryzae) oder gegen den Erreger der Reisstengelkrankheit (Pellicularia sasakii) oder zur Bekämpfung von Krankheiten im Weinbau, wie beispielsweise gegen den Erreger des Mehltaus an Weinreben (Uncinula necator) einsetzen.

Darüber hinaus ist eine breite in-vitro-Wirkung hervorzuheben.

Daneben eignen sich die Wirkstoffe bei guter Pflanzenverträglichkeit und günstiger Warmblütertoxizität auch zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus. Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis,

Schistocerca gregaria. Aus der Ordnung der Dermaptera z.B. Forficula auricularia.

Aus der Ordnung der Isoptera z.B. Reticulitermes spp.. Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta

nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Dabei lassen sich die erfindungsgemäßen Wirkstoffe mit besonders gutem Erfolg zur Bekämpfung von pflanzenschädigenden Insekten, welche das Blattwerk befallen, wie beispielsweise gegen die Raupen der Kohlschabe (Plutella maculipennis) oder gegen die Larven der grünen Reiszikade (Nephotettix cincticeps) einsetzen.

Die Wirkstoffe können dabei in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Bei der Anwendung als Fungizide können die erfindungsgemäßen Wirkstoffe in den Formulierungen in Mischung mit anderen bekannten Wirkstoffen vorliegen wie Fungizide, Insektizide, Akarizide und Herbizide sowie in Mischungen mit Düngemitteln und Wachstumsregulatoren.

Die Wirkstoffe konnen dabei als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich. Bei der Anwendung als Insektizide können die erfindungsgemäßen Wirkstoffe in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können dabei ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Herstellungsbeispiele:

Beispiel 1:

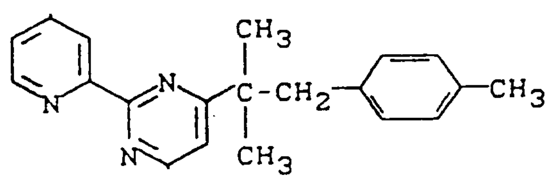

6,3 g (0,04 Mol) 2-Pyridylamidin-hydrochlorid, 9,8 g (0,04 Mol) 1-Dimethylamino-4,4-dimethyl-5-(4-methylphenyl)-pent-1-en-3-on und 10,7 g (0,05 Mol) Natriummethylat werden in 75 ml trockenem Methanol 2 Stunden auf Rückflußtemperatur erhitzt. Nach dem Abkühlen auf Raumtemperatur wird mit Eisessig angesäuert, anschließend im Vakuum eingeengt, der Rückstand mit Wasser verrührt, die wäßrige Lösung abdekantiert, der Rückstand mit Ligroin verrührt, filtriert, das Filtrat im Vakuum eingeengt und der Rückstand chromatographisch gereinigt (Kieselgel; Laufmittel: Essigester).

Man erhält 8,0 g (66% der Theorie) an 2-(2-Pyridyl)-4-[1,1-dimethyl-2-(4-methylphenyl)-ethyl]-pyrimidin als hochviskoses Öl.

$^1H$-NMR ($CDCl_3$/Tetramethylsilan): $\delta$ = 8,8 (d, 1H, J = 6 Hz) ppm.

In entsprechender Weise und gemäß den allgemeinen Angaben zur Herstellung erhält man die folgenden substituierten Pyridylpyrimidine der Formel (I):

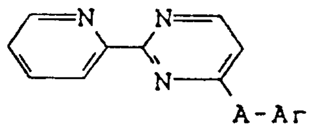

(I)

| Bsp.-Nr. | A | Ar | Physikalische Eigenschaften |
|---|---|---|---|
| 2 | 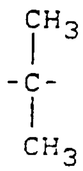 | 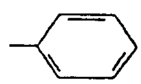 | $^1H$-NMR*): 8.8 (d, 1H, J = 6 Hz) |
| 3 | 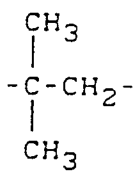 | 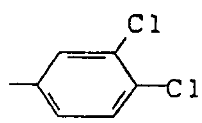 | $^1H$-NMR*): 8.85 (d, 1H, J = 6 Hz) |
| 4 | 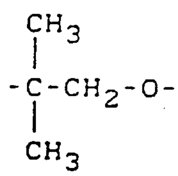 | 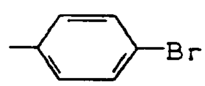 | $^1H$-NMR*): 8.9 (d, 1H, J = 6 Hz) |

| Bsp.-Nr. | A | Ar | Physikalische Eigenschaften |
|---|---|---|---|
| 5 | 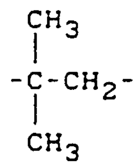 | 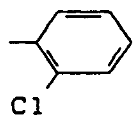 | $^{1}H$-NMR*): 8.8 (d, 1H, J = 6 Hz) |
| 6 | 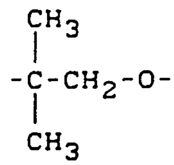 | 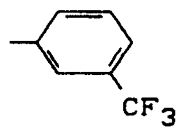 | $^{1}H$-NMR*): 8.9 (d, 1H, J = 6 Hz) |
| 7 | 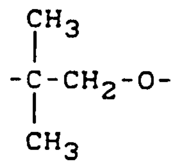 | 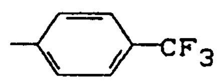 | $^{1}H$-NMR*): 8.85 (d, 1H, J = 6 Hz) |
| 8 | 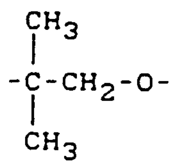 | 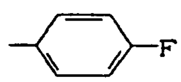 | $^{1}H$-NMR*): 8.9 (d, 1H, J = 6 Hz) |
| 9 | 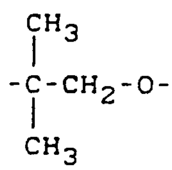 | 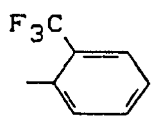 | $^{1}H$-NMR*): 8.8 (d, 1H, J = 6 Hz) |
| 10 | 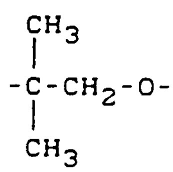 | 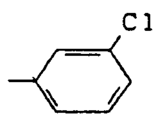 | $^{1}H$-NMR*): 8.85 (d, 1H, J = 6 Hz) |
| 11 | 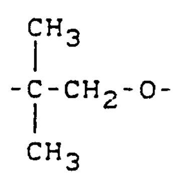 | 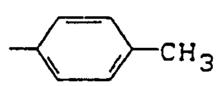 | Fp.: 91-93° C |
| 12 | 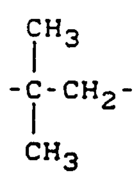 | 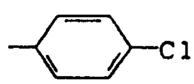 | $^{1}H$-NMR*): 8.8 (d, 1H, J = 6 Hz) |

| Bsp.-Nr. | A | Ar | Physikalische Eigenschaften |
|---|---|---|---|
| 13 | 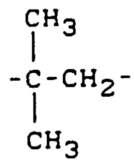 | 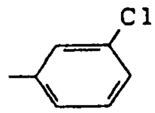 | $^1$H-NMR*): 8.9 (d, 1H, J = 6 Hz) |
| 14 | 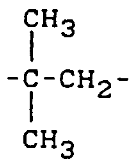 | 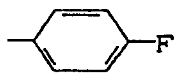 | $^1$H-NMR*): 8.9 (d, 1H, J = 6 Hz) |
| 15 | 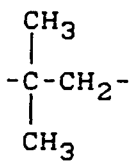 | 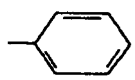 | $^1$H-NMR*): 8.8 (d, 1H, J = 6 Hz) |
| 16 | 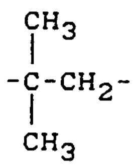 | 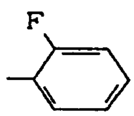 | $^1$H-NMR*): 8.85 (d, 1H, J = 6 Hz) |
| 17 | 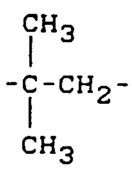 | 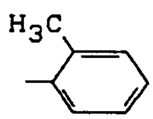 | $^1$H-NMR*): 8.8 (d, 1H, J = 6 Hz) |
| 18 | 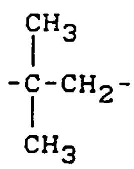 | 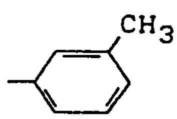 | $^1$H-NMR*): 8.8 (d, 1H, J = 6 Hz) |
| 19 | 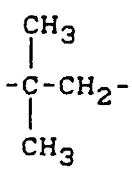 | 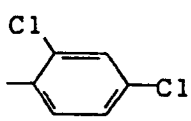 | $^1$H-NMR*): 8.85 (d, 1H, J = 6 Hz) |
| 20 | 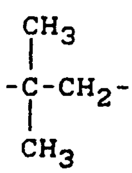 | 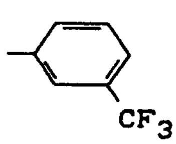 | $^1$H-NMR*): $\delta$ = 8.85 (d, 1H; J = 6 Hz) |

| Bsp.-Nr. | A | Ar | Physikalische Eigenschaften |
|---|---|---|---|
| 21 | 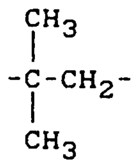 | 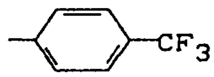 | $^1H$-NMR*): δ = 8.8 (d, 1H; J = 6 Hz) |
| 22 | 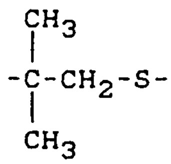 | 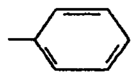 | Fp.: 75-77° C |
| 23 | 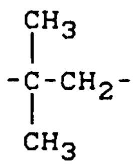 | 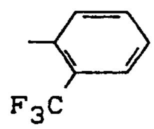 | $^1H$-NMR*): δ = 8.9 (d, 1H; J = 6 Hz) |
| 24 | 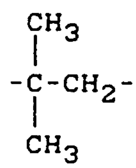 | 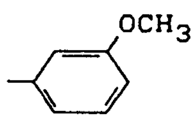 | $^1H$-NMR*): δ = 8.85 (d, 1H; J = 6 Hz) |
| 25 | 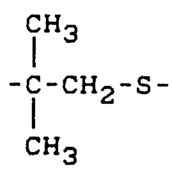 | 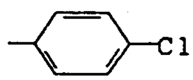 | $^1H$-NMR*): δ = 8.9 (d, 1H; J = 6 Hz) |
| 26 | 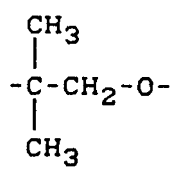 | 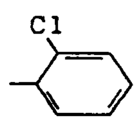 | $^1H$-NMR*): δ = 8.9 (d, 1H; J = 6 Hz) |
| 27 | 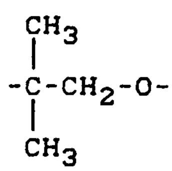 | 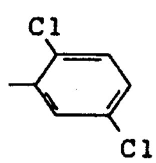 | $^1H$-NMR*): δ = 8.9 (d, 1H; J = 6 Hz) |
| 28 | 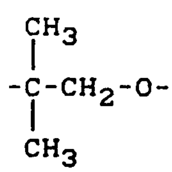 | 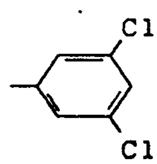 | $^1H$-NMR*): δ = 8.9 (d, 1H; J = 6 Hz) |

| Bsp.-Nr. | A | Ar | Physikalische Eigenschaften |
|---|---|---|---|
| 29 | 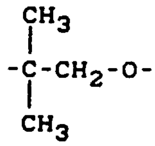 | 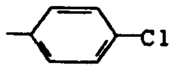 | $^1$H-NMR*): δ = 9.0 (d, 1H; J = 6 Hz) |
| 30 | 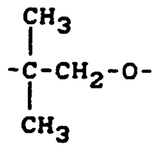 | 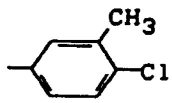 | $^1$H-NMR*): δ = 8.95 (d, 1H; J = 6 Hz) |
| 31 | 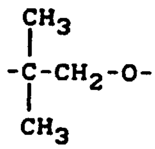 | 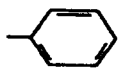 | $^1$H-NMR*): δ = 8.95 (d, 1H; J = 6 Hz) |
| 32 | 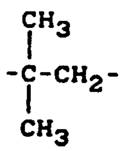 | 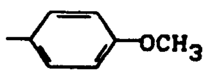 | $^1$H-NMR*): δ = 8.9 (d, 1H; J = 6 Hz) |
| 33 | 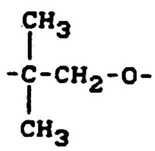 | 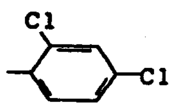 | $^1$H-NMR*): δ = 8.9 (d, 1H; J = 6 Hz) |
| 34 | 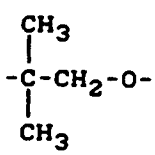 | 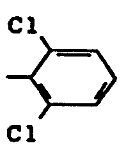 | $^1$H-NMR*): δ = 8.9 (d, 1H; J = 6 Hz) |
| 35 | 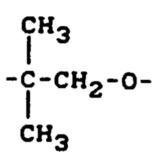 | 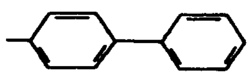 | $^1$H-NMR*): δ = 8.9 (d, 1H; J = 6 Hz) |
| 36 | 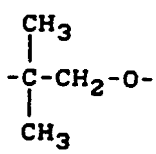 | 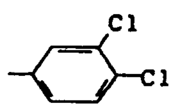 | $^1$H-NMR*): δ = 8.95 (d, 1H; J = 6 Hz) |
| 37 | 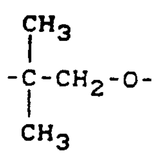 | 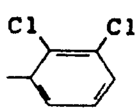 | $^1$H-NMR*): δ = 8.95 (d, 1H; J = 6 Hz) |

| Bsp.-Nr. | A | Ar | Physikalische Eigenschaften |
|---|---|---|---|
| 38 | 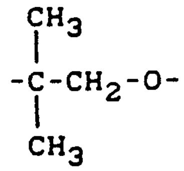 | 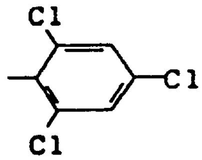 | $^1$H-NMR*): $\delta$ = 8.9 (d, 1H; J = 6 Hz) |
| 39 | 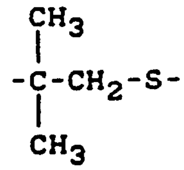 | 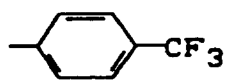 | $^1$H-NMR*): $\delta$ = 8.9 (d, 1H; J = 6 Hz) |

*) Die $^1$H-NMR-Spektren wurden in Deuterochloroform ($CDCl_3$) oder Hexadeuterodimethylsulfoxid (DMSO-$d_6$) mit Tetramethylsilan (TMS) als innerem Standard aufgenommen. Angegeben ist die chemische Verschiebung als $\delta$-Wert in ppm.

Beispiel III-1:

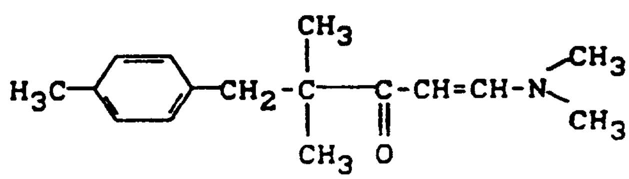

33,3 g (0,175 Mol) 4-(4-Methylphenyl)-3,3-dimethylbutan-2-on und 22,9 g (0,193 Mol) Dimethylformamiddimethylacetal werden zusammengegeben und 2 Stunden lang auf Rückflußtemperatur erhitzt. Nach dem Abkühlen auf Raumtemperatur wird im Vakuum eingeengt, der Rückstand mit Ligroin verrührt, abgesaugt und getrocknet.

Man erhält 14,7 g (34% der Theorie) an 1-Dimethylamino-4,4-dimethyl-5-(4-methylphenyl)-pent-1-en-3-on vom Schmelzpunkt 74 bis 76 °C.

## Herstellung der Ausgangsverbindung:

Beispiel IV-1:

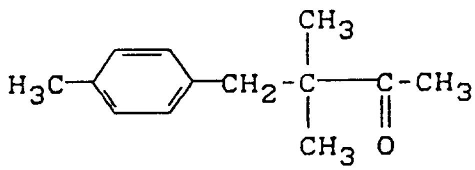

Zu 84 g (1,5 Mol) gepulvertem Kaliumhydroxid und 4,8 g (0,015 Mol) Tetrabutylammoniumbromid in 125 ml trockenem Toluol gibt man bei Raumtemperatur tropfenweise unter Rühren zunächst 51,6 g (0,6 Mol) Methyl-isopropyl-keton und anschließend 70,3 g (0,5 ml) 4-Methylbenzylchlorid in 125 ml trockenem Toluol und rührt dann bei Raumtemperatur 16 Stunden. Zur Aufarbeitung wird die Reaktionsmischung mit Wasser versetzt, die organische Phase abgetrennt, über Natriumsulfat getrocknet, eingeengt und im Vakuum destilliert.

Man erhält 36,1 g (38% der Theorie) an 3,3-Dimethyl-4-(4-methylphenyl)-butan-2-on vom Siedepunkt 98 bis 101 °C bei 5 mbar.

In entsprechender Weise und gemäß den allgemeinen Angaben zur Herstellung erhält man die folgenden Enamine der Formel (III):

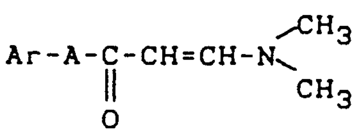

(III)

| Bsp.-Nr. | Ar | A | Physikalische Eigenschaften |
|---|---|---|---|
| III-2 | 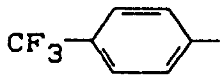 | 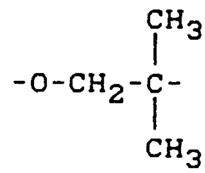 | $^1$H-NMR*): 5.25 (d, 1H, J = 12 Hz) |
| III-3 | 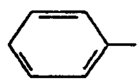 | 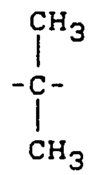 | Fp: 63-66$^o$C |
| III-4 | 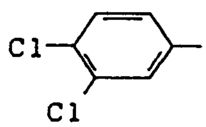 | 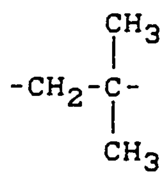 | $^1$H-NMR*): 5.25 (d, 1H, J = 12 Hz) |
| III-5 | 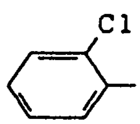 | 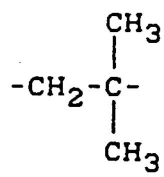 | $^1$H-NMR*): 5.25 (d, 1H, J = 12 Hz) |
| III-6 | 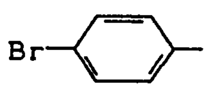 | 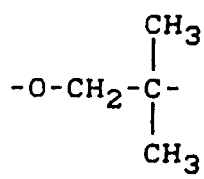 | $^1$H-NMR*): 5.3 (d, 1H, J = 12 Hz) |

| Bsp.-Nr. | Ar | A | Physikalische Eigenschaften |
|---|---|---|---|
| III-7 | 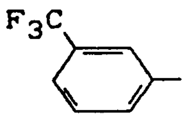 | 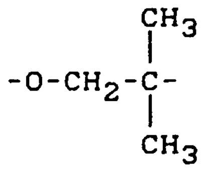 | $^1$H-NMR*): 5.25 (d, 1H, J = 12 Hz) |
| III-8 | 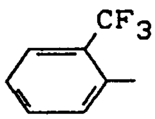 | 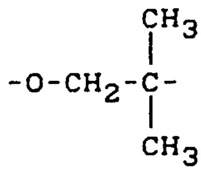 | $^1$H-NMR*): 5.3 (d, 1H, J = 12 Hz) |
| III-9 | 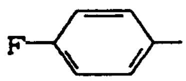 | 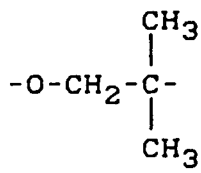 | $^1$H-NMR*): 5.25 (d, 1H, J = 12 Hz) |
| III-10 | 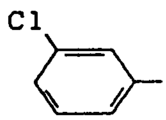 | 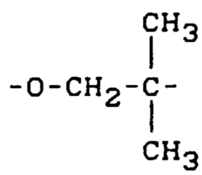 | $^1$H-NMR*): 5.25 (d, 1H, J = 12 Hz) |
| III-11 | 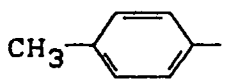 | 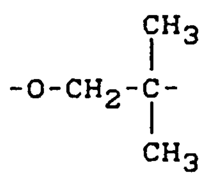 | $^1$H-NMR*): 5.25 (d, 1H, J = 12 Hz) |
| III-12 | 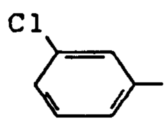 | 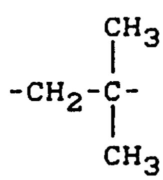 | Fp.: 99-101°C |
| III-13 | 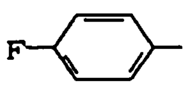 | 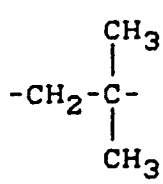 | Fp.: 79-81°C |

| Bsp.-Nr. | Ar | A | Physikalische Eigenschaften |
|---|---|---|---|
| III-14 | 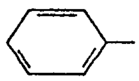 | 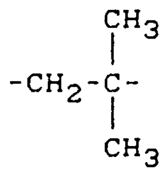 | Fp.: 54-56° C |
| III-15 | 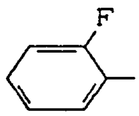 | 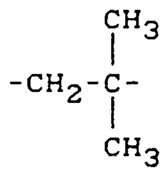 | $^1$H-NMR*): 5.25 (d, 1H, J = 12 Hz) |
| III-16 | 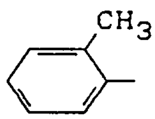 | 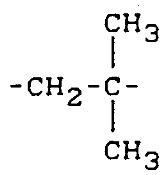 | $^1$H-NMR*): 5.2 (d, 1H, J = 12 Hz) |
| III-17 | 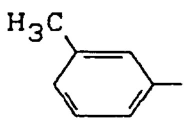 | 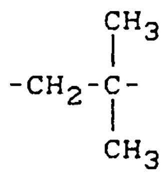 | $^1$H-NMR*): 5.2 (d, 1H, J = 12 Hz) |
| III-18 | 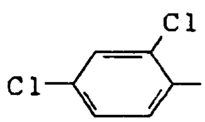 | 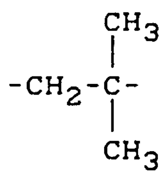 | $^1$H-NMR*): 5.2 (d, 1H, J = 12 Hz) |
| III-19 | 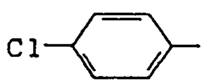 | 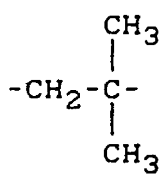 | $^1$H-NMR*): 5.2 (d, 1H, J = 12 Hz) |
| III-20 | 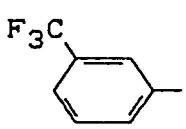 | 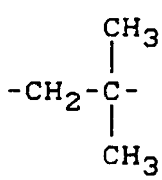 | $^1$H-NMR*): $\delta$ = 5.2 (d, 1H; J = 12 Hz) |

| Bsp.-Nr. | Ar | A | Physikalische Eigenschaften |
|---|---|---|---|
| III-21 | 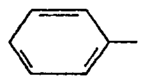 | 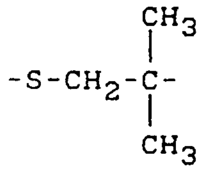 | $^1$H-NMR*): $\delta$ = 5.2 (d, 1H; J = 12 Hz) |
| III-22 | 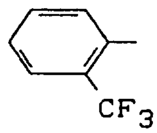 | 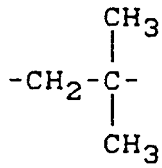 | $^1$H-NMR*): $\delta$ = 5.3 (d, 1H; J = 12 Hz) |
| III-23 | 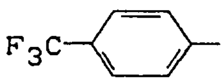 | 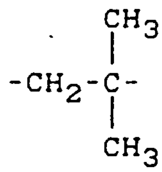 | Fp.: 121-123° C |
| III-24 | 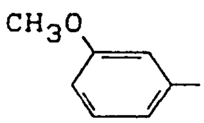 | 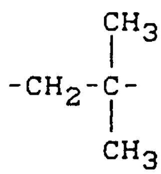 | $^1$H-NMR*): $\delta$ = 5.25 (d, 1H; J = 12 Hz) |
| III-25 | 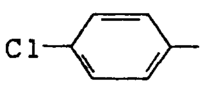 | 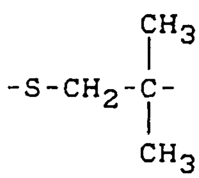 | $^1$H-NMR*): $\delta$ = 5.2 (d, 1H; J = 12 Hz) |
| III-26 | 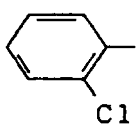 | 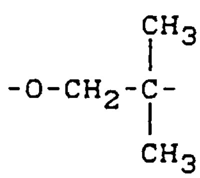 | $^1$H-NMR*): $\delta$ = 5.35 (d, 1H; J = 12 Hz) |
| III-27 | 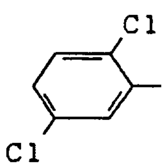 | 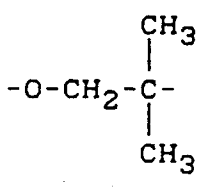 | Fp.: 99-102° C |

| Bsp.-Nr. | Ar | A | Physikalische Eigenschaften |
|---|---|---|---|
| III-28 | 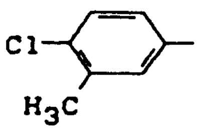 | 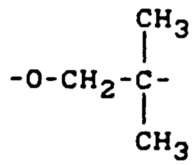 | $^1$H-NMR*): δ = 5.25 (d, 1H; J = 12 Hz) |
| III-29 | 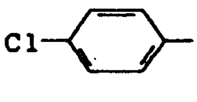 | 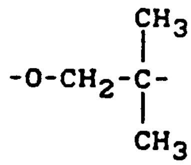 | $^1$H-NMR*): δ = 5.25 (d, 1H; J = 12 Hz) |
| III-30 | 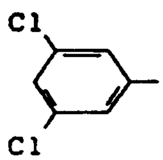 | 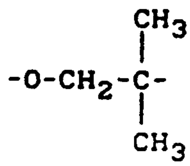 | $^1$H-NMR*): δ = 5.2 (d, 1H; J = 12 Hz) |
| III-31 | 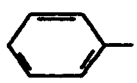 | 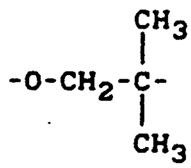 | $^1$H-NMR*): δ = 5.3 (d, 1H; J = 12 Hz) |
| III-32 | 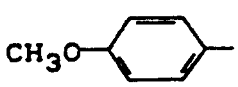 | 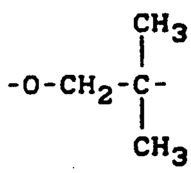 | $^1$H-NMR*): δ = 5.2 (d, 1H; J = 12 Hz) |
| III-33 | 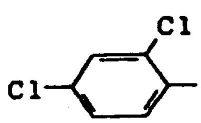 | 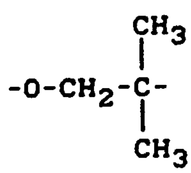 | Fp.: 71-74° C |
| III-34 | 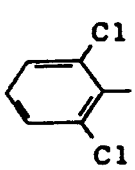 | 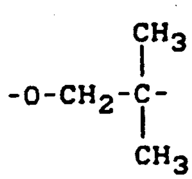 | $^1$H-NMR*): δ = 5.35 (d, 1H; J = 12 Hz) |

| Bsp.-Nr. | Ar | A | Physikalische Eigenschaften |
|---|---|---|---|
| III-35 | 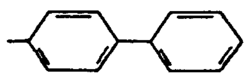 | 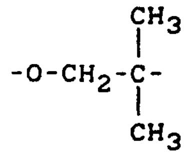 | $^1$H-NMR*): δ = 5.3 (d, 1H; J = 12 Hz) |
| III-36 | 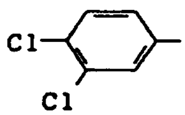 | 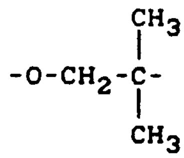 | $^1$H-NMR*): δ = 5.2 (d, 1H; J = 12 Hz) |
| III-37 | 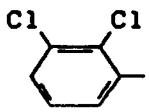 | 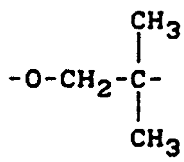 | $^1$H-NMR*): δ = 5.3 (d, 1H; J = 12 Hz) |
| III-38 | 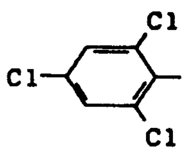 | 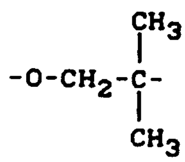 | $^1$H-NMR*): δ = 5.32 (d, 1H; J = 12 Hz) |
| III-39 | 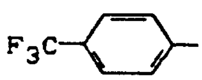 | 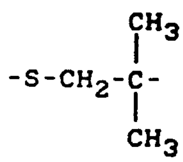 | $^1$H-NMR*): δ = 5.2 (d, 1H; J = 12 Hz) |
| III-40 | 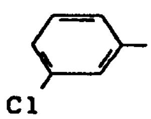 | 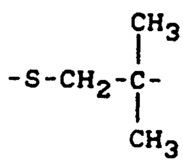 | $^1$H-NMR*): δ = 5.2 (d, 1H; J = 12 Hz) |
| III-41 | 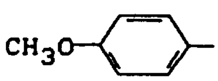 | 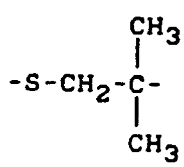 | $^1$H-NMR*): δ = 5.2 (d, 1H; J = 12 Hz |
| III-42 | 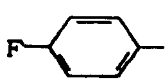 | 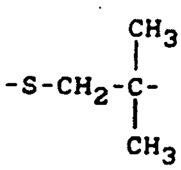 | $^1$H-NMR*): δ = 5.2 (d, 1H; J = 12 Hz) |

| Bsp.-Nr. | Ar | A | Physikalische Eigenschaften |
|---|---|---|---|
| III-43 | 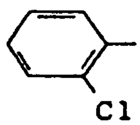 | 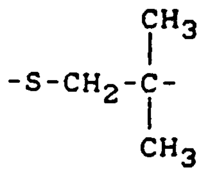 | $^1H$-NMR*): $\delta$ = 5.2 (d, 1H; J = 12 Hz) |

*) Die $^1H$-NMR-Spektren wurden in Deuterochloroform ($CDCl_3$) mit Tetramethylsilan (TMS) als innerem Standard aufgenommen. Angegeben ist die chemische Verschiebung als $\delta$-Wert in ppm.

Anwendungsbeispiele:

In den folgenden Anwendungsbeispielen wurden die nachstehend aufgeführten Verbindungen als Vergleichssubstanzen eingesetzt:

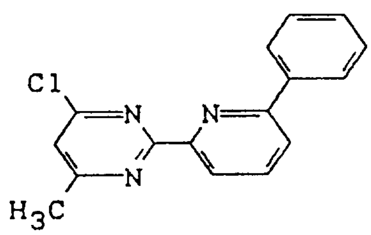

(A)

2-(6-Phenyl-2-pyridyl)-4-chlor-6-methyl-pyrimidin (bekannt aus EP-A 259 139)

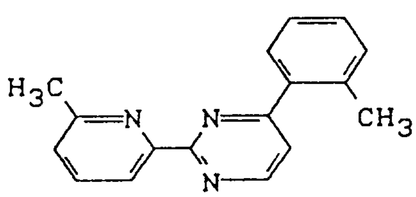

(B)

2-(6-Methyl-2-pyridyl)-4-(2-methylphenyl)-pyrimidin (bekannt aus EP-A 270 362)

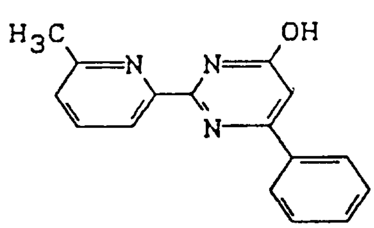

(C)

2-(6-Methyl-2-pyridyl)-4-hydroxy-6-phenyl-pyrimidin (bekannt aus EP-A 270 362)

Beispiel A

Cochliobolus sativus-Test (Gerste)/protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Cochliobolus sativus besprüht. Die Pflanzen verbleiben 48 Stunden bei 20 °C und 100 % rel. Luftfeuchtigkeit in einer Inkubationskabine.

Die Pflanzen werden in einem Gewächshaus bei einer Temperatur von ca. 20 °C und einer relativen Luftfeuchtigkeit von ca. 80 % aufgestellt.

7 Tage nach der Inokulation erfolgt die Auswertung.

Die Verbindungen gemäß den Herstellungsbeispielen 5, 6, 8, 9, 10 und 11 zeigen bei einer Wirkstoffkonzentration in der Spritzbrühe von 250 ppm einen Wirkungsgrad von 81 - 100 %. Die Vergleichsbindungen (A), (B) und (C) zeigen bei gleicher Wirkstoffkonzentration keine Wirkung.

Beispiel B

Erysiphe-Test (Weizen) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.tutici bestäubt.

Die Pflanzen werden in einem Gewächshaus bei einer Temperatur von ca. 20 °C und einer relativen Luftfeuchtigkeit von ca. 80 % aufgestellt, um die Entwicklung von Mehltaupusteln zu begünstigen.

7 Tage nach der Inokulation erfolgt die Auswertung.

Die Verbindungen gemäß den Herstellungsbeispielen 2 und 7 zeigen bei einer Wirkstoffkonzentration in der Spritzbrühe von 250 ppm einen Wirkungsgrad von 100 %. Die Vergleichsverbindung (B) zeigt keine Wirkung.

Beispiel C

Uncinula-Test (Rebe) / protektiv

Lösungsmittel: 4,7 Gewichtsteile Aceton
Emulgator : 0,3 Gewichtsteile Alkyl-Aryl-Polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Uncinula necator bestäubt.

Die Pflanzen werden anschließend bei 23 bis 24 °C und bei einer relativen Luftfeuchtigkeit von ca. 75% im Gewächshaus aufgestellt.

14 Tage nach der Inokulation erfolgt die Auswertung.

Die Verbindungen gemäß den Herstellungsbeispielen 1, 4, 5, 7, 8, 9, 10, 12, 13, 14, 15, 16, 17, 18 und 19 zeigen bei einer Wirkstoffkonzentration in der Spritzbrühe von 10 ppm einen Wirkungsgrad von 82 - 100 %. Die Vergleichsverbindung (A) zeigt bei gleicher Wirkstoffkonzentration einen Wirkungsgrad von 69 %.

Beispiel D

Plutella-Test

Lösungsmittel: 7 Gewichtsteile Dimethylformamid
Emulgator : 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100%, daß alle Raupen abgetötet wurden; 0% bedeutet, daß keine Raupen abgetötet wurden.

Bei diesem Test zeigt z.B. die Verbindung (2) der Herstellungsbeispiele bei einer Wirkstoffkonzentration von 0,1 % einen Abtötungsgrad von 100 %.

Beispiel E

Nephotettix-Test

Lösungsmittel: 7 Gewichtsteile Dimethylformamid
Emulgator : 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Reiskeimlinge (Oryza sativa) werden durch Tauchen in die Wirkstoffzubereitung der gewünschten Konzentration behandelt und mit Larven der Grünen Reiszikade (Nephotettix cincticeps) besetzt, solange die Keimlinge noch feucht sind.

Nach der gewünschten Zeit wird die Abtötung in % bestimmt, Dabei bedeutet 100%, daß alle Zikaden abgetötet wurden; 0% bedeutet, daß keine Zikaden abgetötet wurden.

Bei diesem Test zeigt z.B. die Verbindung (2) der Herstellungsbeispiele bei einer Wirkstoffkonzentration von 0,1 % einen Abtötungsgrad von 100 %.

## Patentansprüche

**1.** Substituierte Pyridylpyrimidine der Formel

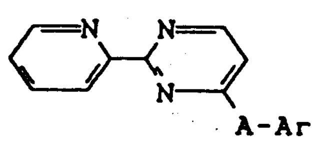

(I)

in welcher

A für einen Rest der Formel

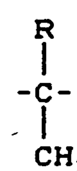 ; 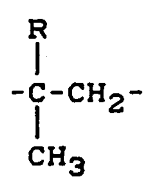 oder 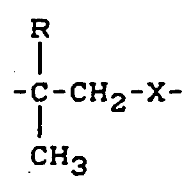

steht, wobei

R jeweils für geradkettiges oder verzweigtes Alkyl mit 1 bis 4 Kohlenstoffatomen steht,

X für Sauerstoff oder Schwefel steht und

Ar für gegebenenfalls einfach bis fünffach, gleich oder verschieden substituiertes Phenyl steht, wobei als Substituenten infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen, oder gegebenenfalls einfach bis fünffach, gleich oder verschieden durch Halogen und/oder geradkettiges oder verzweigtes Alkyl mit 1 bis 4 Kohlenstoffatomen substituiertes Phenyl.

**2.** Substituierte Pyridylpyrimidine der Formel (I) gemäß Anspruch 1, bei welchen

A für einen Rest der Formel

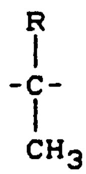 ; 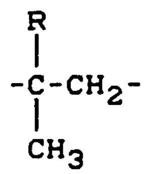 oder 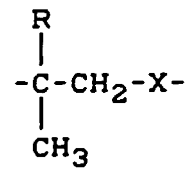

steht,

wobei

R jeweils für Methyl oder Ethyl steht,

X für Sauerstoff oder Schwefel steht und

Ar für gegebenenfalls ein- bis dreifach, gleich oder verschieden substituiertes Phenyl steht, wobei als Substituenten infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy, Trifluormethylthio, Methoxycarbonyl, Ethoxycarbonyl, Methoximinomethyl, Ethoximinomethyl, Methoximinoethyl, Ethoximinoethyl oder gegebenenfalls ein- bis dreifach, gleich oder verschieden durch Fluor, Chlor, Brom, Methyl und/oder Ethyl substituiertes Phenyl.

**3.** Verfahren zur Herstellung von substituierten Pyridylpyrimidinen der Formel (I) gemäß Anspruch 1, dadurch gekennzeichnet, daß man 2-Pyridylamidin-Salze der Formel

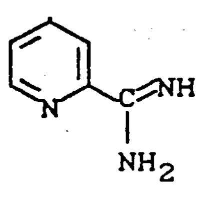 x HY (II)

in welcher

Y für ein Halogenid- oder Hydrogen-sulfatanion steht,

mit Enaminen der Formel

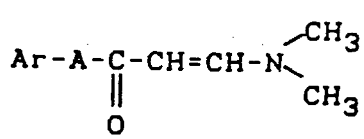 (III)

in welcher

Ar und A die oben angegebenen Bedeutungen haben, gegebenenfalls in Gegenwart eines Verdünnungsmittels und gegebenenfalls in Gegenwart eines Reaktionshilfsmittels umsetzt.

**4.** Schädlingsbekämpfungsmittel, gekennzeichnet durch einen Gehalt an mindestens einem substituierten Pyridylpyrimidin der Formel (I) nach Anspruch 1.

**5.** Verwendung von substituierten Pyridylpyrimidinen der Formel (I) nach Anspruch 1 zur Bekämpfung von Schädlingen.

**6.** Verfahren zur Bekämpfung von Schädlingen, dadurch gekennzeichnet, daß man mindestens ein substituiertes Pyridylpyrimidin der Formel (I) nach Anspruch 1 auf Schädlinge und/oder ihren Lebensraum einwirken läßt.

**7.** Enamine der Formel

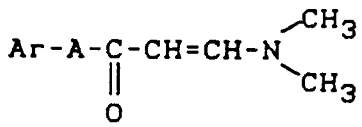 (III)

in welcher

A für einen Rest der Formel

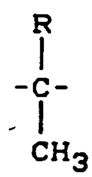 ; 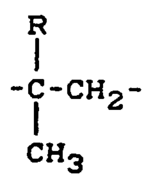 oder 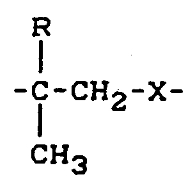

steht,

wobei

R jeweils für geradkettiges oder verzweigtes Alkyl mit 1 bis 4 Kohlenstoffatomen steht,

X für Sauerstoff oder Schwefel steht und

Ar für gegebenenfalls einfach bis fünffach, gleich oder verschieden substituiertes Phenyl steht, wobei als Substituenten infrage kommen: Halogen, Cyano, Nitro, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkoxycarbonyl oder Alkoximinoalkyl mit jeweils 1 bis 4 Kohlenstoffatomen in den einzelnen Alkylteilen, oder gegebenenfalls einfach bis fünffach, gleich oder verschieden durch Halogen und/oder geradkettiges oder verzweigtes Alkyl mit 1 bis 4 Kohlenstoffatomen substituiertes Phenyl.

**8.** Verfahren zur Herstellung von Enaminen der Formel (III) gemäß Anspruch 7, dadurch gekennzeichnet, daß man Ketone der Formel

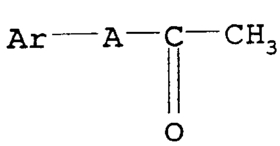 (IV)

in welcher

Ar und A die oben angegebene Bedeutung haben,

mit Formaldehyd-Derivaten der Formel

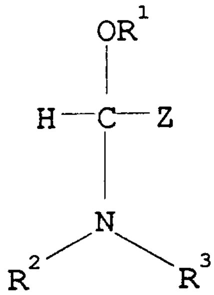

(V)

in welcher

Z für einen Rest $—OR^1$ oder für einen Rest

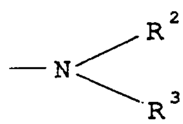

steht und

$R^1$, $R^2$ und $R^3$ unabhängig voneinander jeweils für geradkettiges oder verzweigtes Alkyl mit 1 bis 4 Kohlenstoffatomen stehen,

in annähernd äquimolaren Mengen gegebenenfalls in Gegenwart eines dipolar aprotischen Verdünnungsmittels umsetzt.

**Claims**

1. Substituted pyridylpyrimidines of the formula

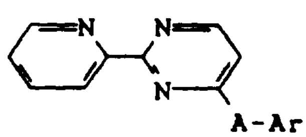

(I)

in which

A represents a radical of the formula

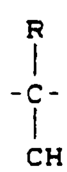

;

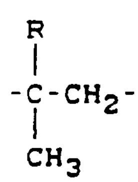

or

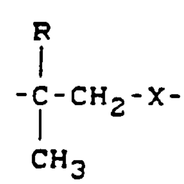

,

where

R in each case represents straight-chain or branched alkyl having 1 to 4 carbon atoms,

X represents oxygen or sulphur and

Ar represents phenyl which is optionally monosubstituted to pentasubstituted by identical or different substituents, suitable substituents being: halogen, cyano, nitro, in each case straight-chain or branched alkyl, alkoxy or alkylthio, in each case having 1 to 4 carbon atoms, in each

case straight-chain or branched halogenoalkyl, halogenoalkoxy or halogenoalkylthio, in each case having 1 to 4 carbon atoms and 1 to 9 identical or different halogen atoms, in each case straight-chain or branched alkoxycarbonyl or alkoxyiminoalkyl, in each case having 1 to 4 carbon atoms in the individual alkyl moieties, or phenyl which is optionally monosubstituted to pentasubstituted by identical or different substituents from the series comprising halogen and/or straight-chain or branched alkyl having 1 to 4 carbon atoms.

**2.** Substituted pyridylpyrimidines of the formula (I) according to Claim 1, in which

A represents a radical of the formula

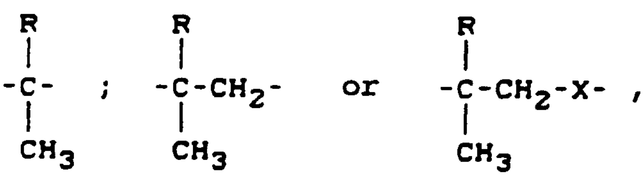

where

R in each case represents methyl or ethyl,

X represents oxygen or sulphur and

Ar represents phenyl which is optionally monosubstituted to trisubstituted by identical or different substituents, suitable substituents being: fluorine, chlorine, bromine, cyano, nitro, methyl, ethyl, n- or i-propyl, n-, i-, s- or t-butyl, methoxy, ethoxy, n- or i-propoxy, methylthio, trifluoromethyl, trifluoromethoxy, trifluoromethylthio, methoxycarbonyl, ethoxycarbonyl, methoxyiminomethyl, ethoxyiminomethyl, methoxyiminoethyl, ethoxyiminoethyl, or phenyl which is optionally monosubstituted to trisubstituted by identical or different substituents from the series comprising fluorine, chlorine, bromine, methyl and/or ethyl.

**3.** Process for the preparation of substituted pyridylpyrimidines of the formula (I) according to Claim 1, characterised in that 2-pyridylamidine salts of the formula

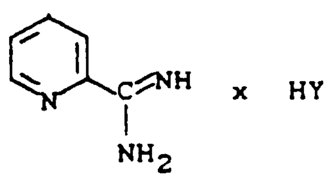

(II)

in which

Y represents a halide anion or a hydrogen sulphate anion,

are reacted with enamines of the formula

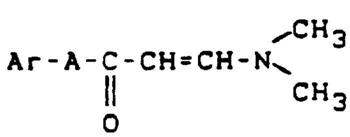

(III)

in which

Ar and A have the abovementioned meanings,

if appropriate in the presence of a diluent and if appropriate in the presence of a reaction auxiliary.

**4.** Pesticides, characterised in that they contain at least one substituted pyridylpyrimidine of the formula (I) according to Claim 1.

**5.** Use of substituted pyridylpyrimidines of the formula (I) according to Claim 1 for combating pests.

**6.** Method of combating pests, characterised in that at least one substituted pyridylpyrimidine of the formula (I) according to Claim 1 is allowed to act on pests and/or their environment.

**7.** Enamines of the formula

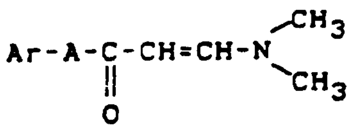

(III)

in which

A represents a radical of the formula

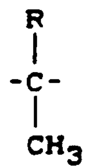 ; 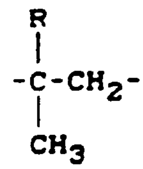 or 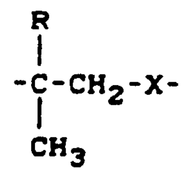 ,

where

R in each case represents straight-chain or branched alkyl having 1 to 4 carbon atoms,

X represents oxygen or sulphur and

Ar represents phenyl which is optionally monosubstituted to pentasubstituted by identical or different substituents, suitable substituents being: halogen, cyano, nitro, in each case straight-chain or branched alkyl, alkoxy or alkylthio, in each case having 1 to 4 carbon atoms, in each case straight-chain or branched halogenoalkyl, halogenoalkoxy or halogenoalkylthio, in each case having 1 to 4 carbon atoms and 1 to 9 identical or different halogen atoms, in each case straight-chain or branched alkoxycarbonyl or alkoxyiminoalkyl, in each case having 1 to 4 carbon atoms in the individual alkyl moieties, or phenyl which is optionally monosubstituted to pentasubstituted by identical or different substituents from the series comprising halogen and/or straight-chain or branched alkyl having 1 to 4 carbon atoms.

**8.** Process for the preparation of enamines of the formula (III) according to Claim 7, characterised in that ketones of the formula

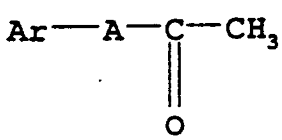

(IV)

in which

Ar and A have the abovementioned meaning,

are reacted with formaldehyde derivatives of the formula

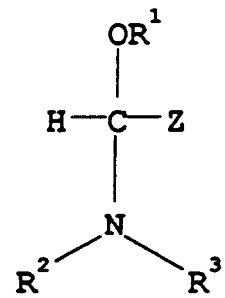

(V)

in which

Z represents a radical $-OR^1$ or a radical

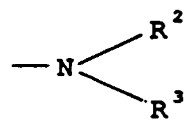

and

$R^1$, $R^2$ and $R^3$ independently of one another in each case represent straight-chain or branched alkyl having 1 to 4 carbon atoms,

in approximately equimolar amounts, if appropriate in the presence of a dipolar aprotic diluent.

## Revendications

**1.** Pyridylpyrimidines substituées, de formule

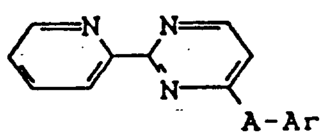

(I)

dans laquelle

A représente un reste de formule

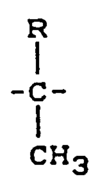

;

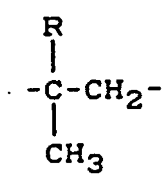

ou

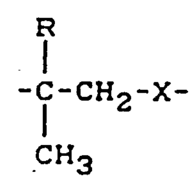

dans laquelle

R représente dans chaque cas un groupe alkyle linéaire ou ramifié ayant 1 à 4 atomes de carbone,

X est de l'oxygène ou du soufre et

Ar représente un groupe phényle portant éventuellement un à cinq substituants identiques ou différents, en considérant comme substituants : un halogène, un groupe cyano, nitro, un groupe alkyle, alkoxy ou alkylthio linéaire ou ramifié avec chacun 1 à 4 atomes de carbone, un groupe halogénalkyle, halogénalkoxy ou halogénalkylthio linéaire ou ramifié ayant chacun 1 à 4 atomes de carbone et 1 à 9 atomes d'halogènes identiques ou différents, un groupe alkoxycarbonyle ou alkoximinoalkyle linéaire ou ramifié ayant chacun 1 à 4 atomes de carbone dans les parties alkyle individuelles, ou un groupe phényle portant éventuellement un à cinq substituants, identiques ou différents, halogéno et/ou alkyle linéaires ou ramifiés ayant

1 à 4 atomes de carbone.

2. Pyridylpyrimidines substituées de formule (I) suivant la revendication 1, dans lesquelles

A représente un reste de formule

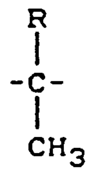 ; 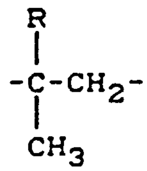 ou 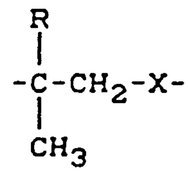

où

R représente à chaque fois un groupe méthyle ou éthyle,

X est de l'oxygène ou du soufre et

Ar représente un groupe phényle portant éventuellement un à trois substituants identiques ou différents, en considérant comme substituants : le fluor, le chlore, le brome, un radical cyano, nitro, méthyle, éthyle, n-propyle, isopropyle, n-butyle, isobutyle, sec.-butyle, tertiobutyle, méthoxy, éthoxy, n-propoxy, isopropoxy, méthylthio, trifluorométhyle, trifluorométhoxy, trifluorméthylthio, méthoxycarbonyle, éthoxycarbonyle, méthoximinométhyle, éthoximinométhyle, méthoximinoéthyle, éthoximinoéthyle ou un groupe phényle éventuellement substitué une ou trois fois, identiques ou différentes, par du fluor, du chlore, du brome, un radical méthyle et/ou éthyle.

3. Procédé de production de pyridylpyrimidines substituées de formule (I) suivant la revendication 1, caractérisé en ce qu'on fait réagir des sels de 2-pyridylamidines de formule

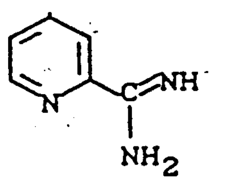 x HY (II)

dans laquelle

Y représente un anion halogénure ou hydrogéno-sulfate,

avec des énamines de formule

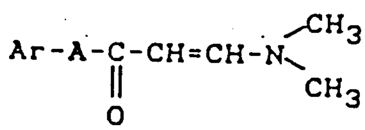 (III)

dans laquelle

Ar et A ont les définitions indiquées ci-dessus,

le cas échéant en présence d'un diluant et en la présence éventuelle d'un agent auxiliaire de réaction.

4. Compositions pesticides, caractérisées par une teneur en au moins une pyridylpyrimidine substituée de formule (I) suivant la revendication 1.

5. Utilisation de pyridylpyrimidines substituées de formule (I) suivant la revendication 1 pour combattre des parasites.

6. Procédé pour combattre des parasites, caractérisé en ce qu'on fait agir sur les parasites et/ou sur leur milieu au moins une pyridylpyrimidine substituée de formule (I) suivant la revendication 1.

**7.** Enamines de formule

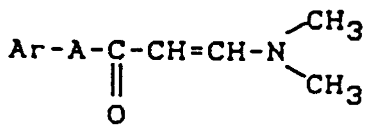

(III)

dans laquelle

A représente un reste de formule

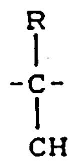

;

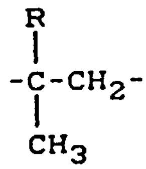

ou

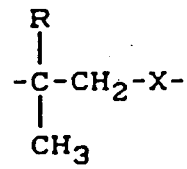

où

R représente dans chaque cas un groupe alkyle linéaire ou ramifié ayant 1 à 4 atomes de carbone,

X est de l'oxygène ou du soufre et

Ar représente un groupe phényle éventuellement substitué une à cinq fois identiques ou différentes, en considérant comme substituants : un halogène, un radical cyano, nitro, un radical alkyle, alkoxy ou alkylthio linéaire ou ramifié ayant chacun 1 à 4 atomes de carbone, un radical halogénalkyle, halogénalkoxy ou halogénalkylthio linéaire ou ramifié ayant chacun 1 à 4 atomes de carbone et 1 à 9 atomes d'halogènes identiques ou différents, un radical alkoxycarbonyle ou alkoximinoalkyle linéaire ou ramifié ayant chacun 1 à 4 atomes de carbone dans les parties alkyle individuelles, ou un radical phényle éventuellement substitué une à cinq fois identiques ou différentes par un halogène et/ou un radical alkyle linéaire ou ramifié ayant 1 à 4 atomes de carbone.

**8.** Procédé de production d'énamines de formule (III) suivant la revendication 7, caractérisé en ce qu'on fait réagir des cétones de formule

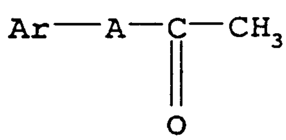

(IV)

dans laquelle

Ar et A ont la définition indiquée ci-dessus,

avec des dérivés de formaldéhyde de formule

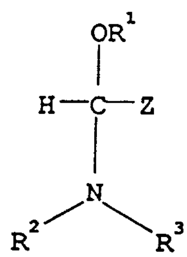

(V)

dans laquelle

Z représente un reste $-OR^1$ ou un reste

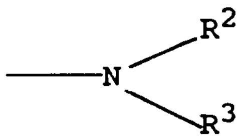

et

$R^1$, $R^2$ et $R^3$ représentent chacun, indépendamment les uns des autres, un groupe alkyle linéaire ou ramifié ayant 1 à 4 atomes de carbone,

en quantités approximativement équimolaires, le cas échéant en présence d'un diluant aprotique dipolaire.